# DEMANDE DE BREVET EUROPEEN

(11) **EP 4 667 049 A1**
(43) Date de publication de la demande: **24.12.2025**
(21) Numéro de dépôt: 25181838.1
(22) Date de dépôt: 10.06.2025
(51) Int. Cl.: A61N 5/06

(54) **DISPOSITIF EMISSIF LUMINEUX A ELEMENT DE GUIDAGE OPTIQUE**

(30) Priorité: 17.06.2024 FR 2406386
(71) Demandeur: Commissariat à l'Energie Atomique et aux Energies Alternatives, 75015 Paris (FR)
(72) Inventeur: RACINE, Benoît, 38054 Grenoble Cedex 09 (FR); BLANQUER, Guillaume, 38054 Grenoble Cedex 09 (FR); PLANAT-CHRETIEN, Anne, 38054 Grenoble Cedex 09 (FR); LAUGIER, Christelle, 38054 Grenoble Cedex 09 (FR)
(74) Mandataire: Cabinet Beaumont

(57) **Abrégé**

La présente description concerne un dispositif émissif lumineux comprenant au moins :
- un élément de guidage optique ;
- une source lumineuse au moins partiellement transparente à au moins une lumière destinée à être émise par la source lumineuse, et configurée pour émettre la lumière au moins du côté d'une première face disposée en regard de l'élément de guidage optique et du côté d'une deuxième face opposée à la première face ;
- une couche réflectrice disposée du côté de la deuxième face de la source lumineuse ;
- un composant optique disposé entre la couche réflectrice et l'élément de guidage optique et au moins partiellement transparent à la lumière destinée à être émise par la source lumineuse ;
et dans lequel la couche réflectrice forme au moins une surface réfléchissante conforme à une surface non plane du composant optique sur laquelle la couche réflectrice est disposée.

## Description

### Domaine technique

La présente description concerne de façon générale les dispositifs émissifs lumineux à élément de guidage optique, ou élément de guidage lumineux.

### Technique antérieure

La photothérapie dynamique, ou PDT, est une technique de traitement consistant à détruire des tissus d'origine tumorale ou non tumorale par l'action conjointe d'un principe actif photosensible injecté dans les tissus et d'une illumination des tissus par une source lumineuse à une longueur d'onde adéquate (dans la gamme du visible) pour permettre la photoactivation du principe actif entraînant la destruction des tissus ciblés par la mort cellulaire. La réaction qui se produit est complexe et implique la présence locale d'oxygène, et donc la présence d'une perfusion locale. L'un des principaux problèmes de cette technique est la faible distance de pénétration de la lumière dans les tissus. En effet, la lumière ne se propage pas suffisamment à l'intérieur de la peau en raison de l'absorption et de la diffusion des photons dans les tissus à traverser, et ne peut donc pas activer correctement les molécules photosensibles injectées dans les tissus et qui sont distribuées de la surface jusqu'à plusieurs centaines de micromètres sous la surface de la peau, en fonction du mode d'injection utilisé. De ce fait, l'efficacité du traitement s'en trouve fortement réduite et seules des pathologies proches de la surface de la peau peuvent être traitées par cette technique.

Afin de contourner ce problème, il est possible d'utiliser une très forte intensité lumineuse et/ou des temps d'exposition plus long (dans la limite des normes réglementaires) pour apporter la quantité d'énergie optique nécessaire à l'activation des molécules photosensibles à l'intérieur des tissus. Mais dans ce cas, il est possible d'endommager la microcirculation des tissus sains voisins, ou la microcirculation des couches superficielles surexposées, ou encore de ne pas atteindre une efficacité du traitement à la profondeur souhaitée. En outre, sous certaines conditions d'éclairement, cette exposition peut provoquer de fortes douleurs (brûlures photochimiques fortes).

Une autre solution au problème de diffusion lumineuse dans les tissus consiste à utiliser des microaiguilles formant des guides lumineux. Ces microaiguilles sont insérées à l'intérieur de la peau et des tissus sur une profondeur légèrement inférieure à la longueur des microaiguilles et par exemple de plusieurs centaines de microns. Grâce à cette configuration, il est possible d'insoler la couche profonde des tissus via le guidage de la lumière réalisé à l'intérieur des microaiguilles, et ainsi diminuer la dose d'irradiation de la peau en surface, tout en atteignant les zones profondes de manière efficace. La lumière peut être introduite dans les microaiguilles par une illumination grand champ ou via des microlentilles disposées au-dessus des microaiguilles. L'utilisation des microlentilles permet de focaliser la lumière directement dans les microaiguilles. Dans cette solution, la lumière est donc dirigée vers les microaiguilles pour une meilleure distribution de la lumière dans la peau. Par contre, l'illumination en surface ne se fait plus, ou de manière beaucoup moins efficace.

Des problèmes analogues de guidage optique peuvent se rencontrer dans d'autres domaines tels que celui des communications optiques, par exemple lorsqu'il s'agit d'optimiser le couplage de la lumière dans un guide d'onde.

### Résumé de l'invention

Il existe un besoin de proposer une solution palliant au moins une partie des inconvénients exposés ci-dessus.

Un mode de réalisation pallie tout ou partie de ces inconvénients et propose un dispositif émissif lumineux comprenant au moins :
- un élément de guidage optique ;
- une source lumineuse au moins partiellement transparente à au moins une lumière destinée à être émise par la source lumineuse, et configurée pour émettre la lumière au moins du côté d'une première face disposée en regard de l'élément de guidage optique et du côté d'une deuxième face opposée à la première face ;
- une couche réflectrice disposée du côté de la deuxième face de la source lumineuse ;
- un composant optique disposé entre la couche réflectrice et l'élément de guidage optique et au moins partiellement transparent à la lumière destinée à être émise par la source lumineuse ;

et dans lequel la couche réflectrice forme au moins une surface réfléchissante conforme à une surface non plane du composant optique sur laquelle la couche réflectrice est disposée.

Selon un mode de réalisation particulier, la source lumineuse comporte au moins une diode électroluminescente organique.

Selon un mode de réalisation particulier, la couche réflectrice est l'une des électrodes de la diode électroluminescente organique.

Selon un mode de réalisation particulier, la surface réfléchissante comporte au moins une partie concave ou convexe.

Selon un mode de réalisation particulier, la surface réfléchissante forme au moins un miroir sphérique ou conique ou hyperbolique.

Selon un mode de réalisation particulier, la couche réflectrice comporte au moins une couche métallique et/ou au moins un miroir de Bragg.

Selon un mode de réalisation particulier, le dispositif émissif lumineux comporte en outre au moins un substrat au moins partiellement transparent à la lumière destinée à être émise par la source lumineuse et disposé entre l'élément de guidage optique et la source lumineuse.

Selon un mode de réalisation particulier, l'élément de guidage optique comporte au moins une microaiguille au moins partiellement transparente à la lumière destinée à être émise par la source lumineuse, ou au moins un guide d'onde.

Selon un mode de réalisation particulier, l'élément de guidage optique comporte une base au moins partiellement transparente à la lumière destinée à être émise par la source lumineuse et plusieurs microaiguilles au moins partiellement transparentes à la lumière destinée à être émise par la source lumineuse et comprenant chacune une première extrémité solidaire de la base, la base étant disposée entre la source lumineuse et les microaiguilles.

Selon un mode de réalisation particulier, l'élément de guidage optique comporte plusieurs microaiguilles au moins partiellement transparentes à la lumière destinée à être émise par la source lumineuse, et dans lequel le composant optique comporte une couche de matériau au moins partiellement transparent à la lumière destinée à être émise par la source lumineuse, la couche de matériau comprenant des creux alignés avec les microaiguilles.

Selon un mode de réalisation particulier, la source lumineuse comporte plusieurs parties distinctes configurées pour émettre des lumières de longueurs d'onde différentes, chacune desdites parties étant disposée en regard d'au moins une des microaiguilles.

Selon un mode de réalisation particulier, la source lumineuse est configurée pour émettre une partie de la lumière entre les microaiguilles.

Selon un mode de réalisation particulier, au moins une partie de la surface réfléchissante est configurée pour réfléchir une partie de la lumière émise du côté de la deuxième face de la source lumineuse entre les microaiguilles.

Il est également proposé un procédé de réalisation d'un dispositif émissif lumineux, comprenant au moins :
- réalisation d'au moins un élément de guidage optique ;
- réalisation d'au moins une source lumineuse au moins partiellement transparente à au moins une lumière destinée à être émise par la source lumineuse, et configurée pour émettre la lumière au moins du côté d'une première face disposée en regard de l'élément de guidage optique et du côté d'une deuxième face opposée à la première face ;
- réalisation d'au moins une couche réflectrice disposée du côté de la deuxième face de la source lumineuse ;
- réalisation d'au moins un composant optique disposé entre la couche réflectrice et l'élément de guidage optique et au moins partiellement transparent à la lumière destinée à être émise par la source lumineuse ;

et dans lequel la couche réflectrice est réalisée telle qu'elle forme au moins une surface réfléchissante conforme à une surface non plane du composant optique sur laquelle la couche réflectrice est disposée.

Selon un mode de réalisation particulier :
- la source lumineuse et la couche réflectrice sont réalisés sur l'élément de guidage optique, ou
- la source lumineuse et la couche réflectrice sont réalisées sur un substrat au moins partiellement transparent à la lumière destinée à être émise par la source lumineuse, le substrat étant solidarisé ensuite à l'élément de guidage optique, ou
- la source lumineuse, la couche réflectrice et le composant optique sont réalisés sur un substrat opaque à la lumière destinée à être émise par la source lumineuse, le composant optique étant solidarisé ensuite à l'élément de guidage optique.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
- la figure 1 représente schématiquement un exemple de dispositif émissif lumineux selon un premier mode de réalisation ;
- la figure 2 représente des exemples de focalisation de lumière obtenue avec différentes formes de surface réfléchissante dans un dispositif émissif lumineux ;
- la figure 3, la figure 4, la figure 5, la figure 6, la figure 7, la figure 8, la figure 9 et la figure 10 représentent des étapes d'un exemple de procédé de réalisation d'un dispositif émissif lumineux selon le premier mode de réalisation ;
- la figure 11 représente schématiquement un exemple de dispositif émissif lumineux selon une variante du premier mode de réalisation ;
- la figure 12 représente schématiquement un exemple de dispositif émissif lumineux selon un deuxième mode de réalisation ;
- la figure 13 représente schématiquement un exemple de dispositif émissif lumineux selon une variante du deuxième mode de réalisation ;
- la figure 14, la figure 15, la figure 16 et la figure 17 représentent des étapes d'un procédé de réalisation d'un dispositif émissif lumineux selon le deuxième mode de réalisation ;
- la figure 18, la figure 19, la figure 20 et la figure 21 représentent des étapes d'un procédé de réalisation d'un dispositif émissif lumineux selon une autre variante du deuxième mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Sur les figures, afin de faciliter leur lecture, les différents éléments et les différentes couches de matériaux ne sont pas représentés à la même échelle les uns par rapport aux autres.

Par souci de clarté, seuls les étapes et éléments utiles à la compréhension des modes de réalisation décrits ont été représentés et sont détaillés.

Sauf précision contraire, lorsque l'on fait référence à deux éléments connectés entre eux, cela signifie directement connectés sans éléments intermédiaires autres que des conducteurs, et lorsque l'on fait référence à deux éléments reliés ou couplés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", "latéral", etc., ou à des qualificatifs d'orientation, tels que les termes "horizontal", "vertical", etc., il est fait référence sauf précision contraire à l'orientation des figures. Toutefois, ces termes ne présument pas de la position et de l'orientation réelles du dispositif lors de son utilisation.

Sauf précision contraire, les expressions "environ", "approximativement", "sensiblement", et "de l'ordre de" signifient à 10 % près, de préférence à 5 % près.

Dans tout le document, l'expression « au moins partiellement transparent » est utilisée pour caractériser le fait qu'un élément puisse être traversé par au moins une partie (par exemple au moins 40 % ou au moins 50 % ou au moins 70 % ou au moins 90 %) de la lumière reçue en entrée de cet élément et/ou émise par cet élément.

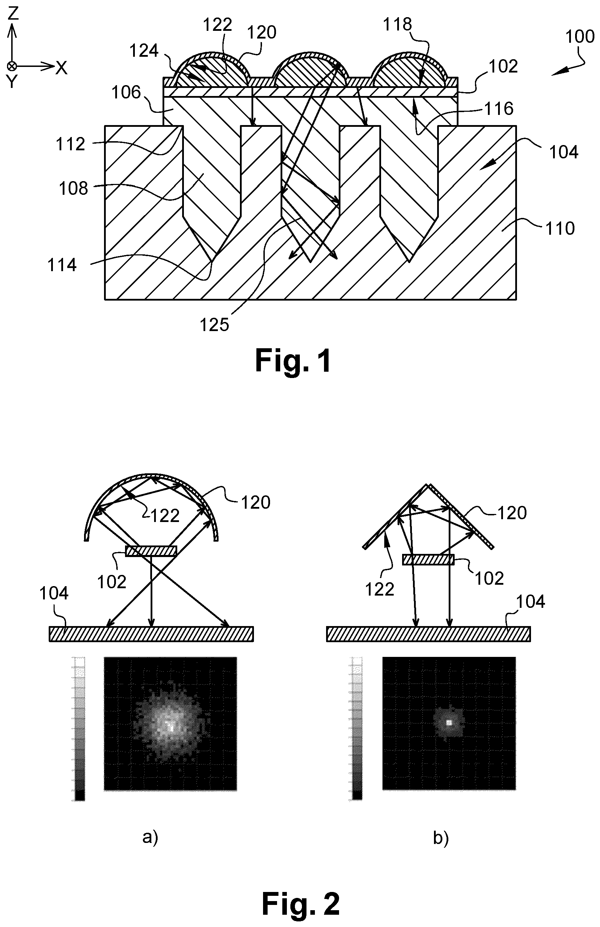

Un exemple de dispositif émissif lumineux 100 selon un premier mode de réalisation est décrit ci-dessous en lien avec la figure 1. Dans cet exemple de réalisation, le dispositif 100 correspond à un dispositif de photothérapie dynamique destiné à réaliser une émission lumineuse à la fois en surface de tissus de peau et également en profondeur dans les tissus, sous la surface extérieure de la peau.

Le dispositif 100 comporte au moins une source lumineuse 102 au moins partiellement transparente (et de préférence transparente) à au moins une lumière destinée à être émise par la source lumineuse 102. Dans l'exemple de réalisation décrit, la source lumineuse 102 est de nature organique et comporte une diode électroluminescente organique, ou OLED (« Organic Light-Emitting Diode » en anglais). Cette OLED comporte des première et deuxième électrodes (l'une correspondant à l'anode de l'OLED et l'autre correspondant à la cathode de l'OLED) à base d'au moins un matériau électriquement conducteur, et au moins une couche émissive à base d'au moins un matériau semi-conducteur organique et disposée entre les première et deuxième électrodes. L'épaisseur (dimension parallèle à l'axe Z sur la figure 1) de l'OLED est par exemple comprise entre 50 nm et 500 nm. A titre d'exemple, les électrodes de l'OLED peuvent comporter au moins l'un des matériaux suivants : Al, Ag, ITO, SnO₂, etc. La couche émissive de l'OLED peut comporter, en fonction de la longueur d'onde souhaitée, au moins l'un des types de matériaux suivants : Irppy, TADF (« thermally activated delayed fluorescent » en anglais, ou à fluorescence retardée activée thermiquement), MR-TADF (« multiple resonance thermally activated delayed fluorescent » en anglais, ou à fluorescence retardée activée thermiquement à multirésonance). En outre, la source lumineuse 102 est configurée pour émettre de la lumière depuis au moins deux faces opposées.

Une source lumineuse 102 comprenant au moins une OLED utilisée dans le dispositif 100 a pour avantage, par rapport à d'autres types de source lumineuse, de réaliser une émission lumineuse homogène, isotrope et sur une surface importante. En variante, le dispositif 100 peut comporter d'autres types de source lumineuse 102 configurée pour émettre depuis deux côtés opposés et qui soit au moins partiellement transparente à la lumière émise.

Le dispositif 100 comporte en outre au moins un élément de guidage optique 104. Dans l'exemple de réalisation décrit, l'élément de guidage optique 104 comporte une base 106 au moins partiellement transparente (et de préférence transparente) à la lumière destinée à être émise par la source lumineuse 102, ainsi que plusieurs microaiguilles 108 également au moins partiellement transparente (et de préférence transparente) à la lumière destinée à être émise par la source lumineuse 102 et destinées à être insérées dans des tissus 110 correspondant à des couches superficielles de la peau. Chacune des microaiguilles 108 comporte une première extrémité 112 solidaire de la base 106 et une deuxième extrémité 114 en forme de pointe. Plus particulièrement, dans l'exemple décrit, chaque microaiguille 108 comporte une partie cylindrique s'étendant depuis la première extrémité 112 et se prolongeant par une partie conique jusqu'à la deuxième extrémité 114. En variante, des formes de microaiguilles 108 autres que celle décrite ci-dessus sont possibles. Par exemple, la section des microaiguilles 108 peut être d'une forme autre qu'un disque. En outre, des formes autres qu'une pointe sont envisageables, par exemple pour orienter l'élément de guidage optique 104 ou le coupler avec un diffuseur local disposé au bout des microaiguilles 108.

Dans l'exemple de réalisation décrit, les microaiguilles 108 permettent de guider la lumière reçue au niveau de la surface de la base 106 sur laquelle est disposée la source lumineuse 102 dans les couches plus profondes de la peau dans laquelle les microaiguilles 108 sont insérées. La forme conique des pointes des microaiguilles 108 permet de bien faire pénétrer les microaiguilles 108 dans la peau et également d'assurer une bonne diffusion de la lumière guidée dans la partie cylindrique des microaiguilles 108. A titre d'exemple, la hauteur de chacune des microaiguilles 108 (dimension parallèle à l'axe Z sur la figure 1) peut être comprise entre 100 µm et 3 mm. La section de la partie cylindrique de chacune des microaiguilles 108, dans un plan perpendiculaire à leur hauteur (plan parallèle au plan (X,Y) sur l'exemple de la figure 1) a par exemple un diamètre compris entre 50 µm et 900 µm. Le pas des microaiguilles 108, c'est-à-dire la distance séparant les axes de révolution de deux microaiguilles 108 voisines, peut être compris entre environ 100 µm et plusieurs millimètres. Selon un exemple de réalisation, les microaiguilles 108 peuvent comporter un matériau biocompatible tel que du polyméthacrylate de méthyle ou PMMA, ou du PLGA (poly (acide lactique-co-glycolique)).

Lorsque le dispositif 100 est destiné à des utilisations autres que la photothérapie dynamique, l'élément de guidage optique 104 peut comporter la base 106 à laquelle sont couplés optiquement un ou plusieurs éléments au moins partiellement transparents (et de préférence transparents) à la lumière destinée à être émise par la source lumineuse 102, ce ou ces éléments pouvant ne pas être des microaiguilles.

Ainsi, l'élément de guidage optique 104 peut comporter par exemple au moins une microaiguille, ou au moins un guide d'onde (exemple d'une application autre que la photothérapie dynamique), et l'élément de guidage optique 104 peut comporter ou non la base 106.

La source lumineuse 102 est configurée pour émettre de la lumière au moins du côté d'une première face 116 disposée en regard de l'élément de guidage optique 104 et également du côté d'une deuxième face 118 opposée à la première face 116. Cette émission lumineuse du côté de chacune des faces 116, 118 est due, dans cet exemple de réalisation, au fait que la source lumineuse 102 correspond à une OLED qui émet de la lumière du côté de chacune de ses électrodes (première électrode disposée du côté de la première face 116 et deuxième électrode disposée du côté de la deuxième face 118). En variante, cette émission lumineuse depuis les deux faces 116, 118 de la source lumineuse 102 peut être obtenue en utilisant d'autres types de source lumineuse 102.

Dans l'exemple de réalisation décrit, la première face 116 de la source lumineuse 102 est disposée directement contre l'élément de guidage optique 104, et plus particulièrement contre la base 106 de l'élément de guidage optique 104. En variante, il est possible qu'au moins un élément au moins partiellement transparent, ou de préférence transparent, à la lumière destinée à être émise par la source lumineuse 102, par exemple un substrat de verre ou de tout autre matériau transparent ou semi-transparent, soit interposé entre la source lumineuse 102 et l'élément de guidage optique 104, comme par exemple des bases coniques sur lesquelles sont disposées les microaiguilles, et avec une éventuelle semelle sur laquelle reposent les bases coniques.

Le dispositif 100 comporte en outre au moins une couche réflectrice 120 disposée du côté de la deuxième face 118 de la source lumineuse 102 et formant une surface réfléchissante 122 du côté de la source lumineuse 102. Cette surface 122 est qualifiée de réfléchissante du fait qu'elle est configurée pour réfléchir au moins une partie et, de préférence, la totalité ou la quasi-totalité de la lumière émise par la source lumineuse 102 du côté de sa deuxième face 118. La couche réflectrice 120 comporte par exemple au moins un métal tel que de l'argent ou de l'aluminium. L'épaisseur de la couche réflectrice 120 est par exemple comprise entre 50 nm et 500 nm.

En variante, la couche réflectrice 120 peut comporter au moins un miroir de Bragg configuré pour réfléchir la ou les longueurs d'onde d'intérêt émises par la source lumineuse 102 du côté de sa deuxième face 118, c'est-à-dire la lumière destinée à être envoyée en direction des tissus 110.

Dans tous les cas, les propriétés de la couche réflectrice 120 (matériau(x) utilisé(s), épaisseur, forme, etc.) peuvent être telles que de la surface réfléchissante 122 réfléchisse le plus de lumière possible afin d'avoir une perte lumineuse qui soit la plus faible possible au niveau de cette couche réflectrice 120.

Le dispositif 100 comporte en outre au moins un composant optique 124 disposé entre la couche réflectrice 120 et l'élément de guidage optique 104, et plus particulièrement entre la source lumineuse 102 et la couche réflectrice 120 dans le premier mode de réalisation. Sur l'exemple de la figure 1, le dispositif 100 comporte plusieurs composants optiques 124 chacun disposé en regard de l'une des microaiguilles 108. Le pas (distance entre les centres de deux composants optiques 124 voisins) avec lequel les composants optiques 124 sont réalisés peut être égal à celui des microaiguilles 108. Les composants optiques 124 sont au moins partiellement transparents, et de préférence transparents, à la lumière destinée à être émise par la source lumineuse 102 du côté de sa deuxième face 118. Selon un exemple de réalisation, les composants optiques 124 comportent un polymère de type résine ou un oxyde tel que du SiO₂ ou bien du SiN ou tout autre matériau adapté. En outre, l'épaisseur de chacun des composants optiques 124 (c'est-à-dire leur dimension parallèle à l'axe Z sur l'exemple de la figure 1) est par exemple comprise entre 50 µm et 2 mm.

La couche réflectrice 120 est disposée sur les composants optiques 124 telle que la surface réfléchissante 122 soit conforme à une surface non plane des composants optiques 124 et réalise ainsi une réflexion lumineuse selon une directivité et/ou une focalisation souhaitée. Ainsi, la géométrie de la surface réfléchissante 122 en regard de chaque microaiguille 108 dépend de celle de la surface non plane de chaque composant optique 124. Dans l'exemple de réalisation décrit, chaque composant optique 124 forme une surface concave sur laquelle la couche réflectrice 120 est disposée, cette forme correspondant à celle de la surface réfléchissante 122. Par exemple, les composants optiques 124 peuvent être tels que la surface réfléchissante 122 forme, en regard de chaque microaiguille 108, au moins un miroir sphérique, ou calotte sphérique, ou conique, ou hyperbolique. En variante, chaque composant optique 124 peut avoir une forme convexe ou une autre forme non plane adaptée pour réaliser une réflexion lumineuse souhaitée. Par exemple, les composants optiques 124 peuvent être tels que, combinés à la surface réfléchissante 122, ils permettent d'augmenter localement la directivité de la lumière pour la renvoyer avec un angle adéquat dans l'élément de guidage optique 104.

En outre, les composants optiques 124 combinés à la surface réfléchissante 122 peuvent être configurés pour focaliser la lumière émise par la source lumineuse 102 du côté de la deuxième face 118 dans chacune des microaiguilles 108, comme illustré par les flèches désignées par la référence 125. Le choix de la forme de la surface non plane des composants optiques 124 sur laquelle est disposée la surface réfléchissante 122 de la couche réflectrice 120 peut dépendre de la focalisation souhaitée de la lumière dans l'élément de guidage optique 104, et plus particulièrement dans les microaiguilles 108 dans l'exemple décrit. Sur la figure 2, la vue a) représente la focalisation obtenue sur la surface d'un élément de guidage optique 104 par laquelle la lumière entre, lorsque la surface réfléchissante 122 forme un miroir sphérique, et la vue b) représente la focalisation obtenue lorsque la surface réfléchissante 122 forme un miroir conique. Ces figures montrent que la focalisation obtenue est plus importante lorsque la surface réfléchissante 122 forme un miroir conique que lorsqu'elle forme un miroir sphérique.

Dans le premier mode de réalisation, la source lumineuse 102 est configurée pour émettre, depuis sa première face 116, une partie de la lumière dans les microaiguilles 108 et une autre partie de la lumière entre les microaiguilles 108. La source lumineuse 102 est également configurée pour émettre, depuis sa deuxième face 118, une partie de la lumière dans les microaiguilles 108 après avoir traversé les composants optiques 124, s'être réfléchi sur la surface réfléchissante 122 et avoir traversé de nouveau les composants optiques 124 et la source lumineuse 102, et une autre partie de la lumière entre les microaiguilles 108 après s'être réfléchie sur les parties de la couche réflectrice 120 disposées entre les composants optiques 124 et avoir traversé la source lumineuse 102. En effet, les parties de la deuxième face 118 de la source lumineuse 102 non recouvertes par les composants optiques 124 sont recouvertes directement par la couche réflectrice 120. Ainsi, dans l'exemple de réalisation décrit, cette partie de la lumière envoyée entre les microaiguilles 108 rentre directement dans les tissus 110 depuis la surface extérieure de la peau, tandis que la partie de la lumière envoyée dans les microaiguilles 108 se reflète contre les parois de la partie cylindrique des microaiguilles 108 avant de ressortir dans les tissus 110 au niveau des parties coniques des microaiguilles 108. Ainsi, grâce au dispositif 100, de la lumière est envoyée à la fois en surface des tissus 110 (correspondant à l'irradiation superficielle de la peau) et également à différentes profondeurs des tissus 110. La lumière envoyée dans les tissus 110 par le dispositif 100 n'est donc pas localisée uniquement en surface ou uniquement en profondeur dans les tissus 110. Cela permet, dans le cas d'une utilisation du dispositif 100 pour de la photothérapie dynamique, d'obtenir une efficacité optimale du traitement réalisé, à différentes profondeurs des tissus 110.

En variante du premier mode de réalisation décrit ci-dessus, l'élément de guidage optique 104 peut correspondre à un guide d'onde. Dans ce cas, la couche réflectrice 120 et le ou les composants optiques 124 permettent d'augmenter l'intensité lumineuse envoyée dans ce guide d'onde du fait que la lumière émise depuis la deuxième face 118 de la source lumineuse 102 peut être réfléchie et focalisée en direction du guide d'onde. Le dispositif 100 selon une telle variante peut par exemple être utilisé dans le domaine des communications optiques afin d'optimiser le couplage de la lumière dans le guide d'onde correspondant à l'élément de guidage optique 104.

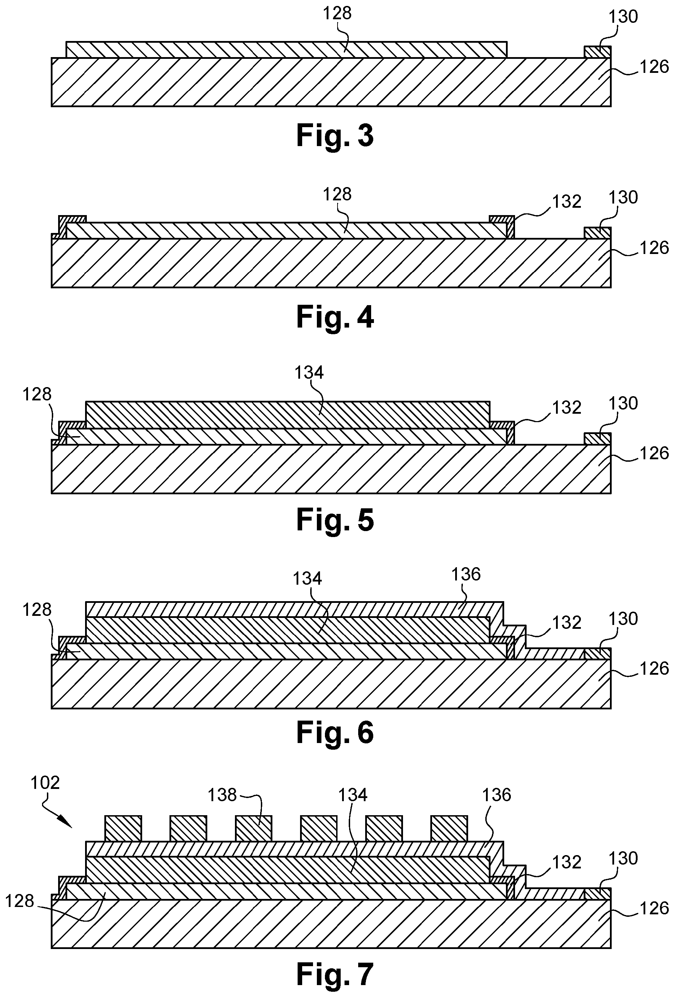

Un exemple de procédé de réalisation du dispositif 100 selon le premier mode de réalisation est décrit ci-dessous en lien avec les figures 3 à 10.

Dans cet exemple, la source lumineuse 102, la couche réflectrice 120 et les composants optiques 124 sont réalisés sur un substrat 126 au moins partiellement transparent à la lumière destinée à être émise par la source lumineuse 102. L'épaisseur du substrat 126 est par exemple égale à quelques centaines de microns. En variante, il est possible que la source lumineuse 102, la couche réflectrice 120 et les composants optiques 124 soient réalisés directement sur l'élément de guidage optique 104, comme c'est le cas sur l'exemple de la figure 1.

Dans l'exemple de réalisation décrit, la source lumineuse 102 réalisée correspond à une OLED. Ainsi, dans cet exemple, une première électrode 128 transparente ou semi-transparente, c'est-à-dire apte à laisser passer au moins une partie de la lumière destinée à être émise depuis la ou les couches émissives de la source lumineuse 102, est réalisée sur le substrat 126. La première électrode 128 correspond par exemple à l'anode de l'OLED formant la source lumineuse 102. Un plot de contact 130 auquel une deuxième électrode de la source lumineuse 102 est destinée à être couplée électriquement est également réalisé sur le substrat 126, à côté de la première électrode 128 (voir figure 3).

Une portion isolante 132, comprenant par exemple de la résine, est ensuite formée, par exemple par dépôt, en périphérie de la première électrode 128 (voir figure 4). Cette portion isolante 132 est destinée à isoler électriquement la première électrode 128 vis-à-vis de la liaison électrique qui sera réalisée entre la deuxième électrode de la source lumineuse 102 et le plot de contact 130.

Une ou plusieurs couches émissives 134, comprenant ici au moins un matériau organique, sont ensuite déposées sur la première électrode 128 (voir figure 5).

Une deuxième électrode 136 transparente ou semi-transparente est réalisée sur la ou les couches émissives 134. Cette deuxième électrode 136 correspond par exemple à la cathode de l'OLED formant la source lumineuse 102. Une partie de cette deuxième électrode 136 est déposée sur au moins un flanc latéral de la ou des couches émissives 134, sur une partie de la portion isolante 132 et sur une partie du substrat 126 de manière à être en contact avec le plot de contact 130 (voir figure 6). A ce stade du procédé, la réalisation de la source lumineuse 102 est achevée.

Bien que non visible, une couche d'encapsulation transparente ou semi-transparente peut être déposée sur la source lumineuse 102.

Les composants optiques 124 sont ensuite réalisés sur la source lumineuse 102. Dans l'exemple de réalisation décrit, des plots 138 du matériau destiné à former les composants optiques 124, par exemple des plots de résine transparente ou semi-transparente, sont réalisés par exemple par dépôt au-dessus de la deuxième électrode 136, par exemple sur la couche d'encapsulation (voir figure 7).

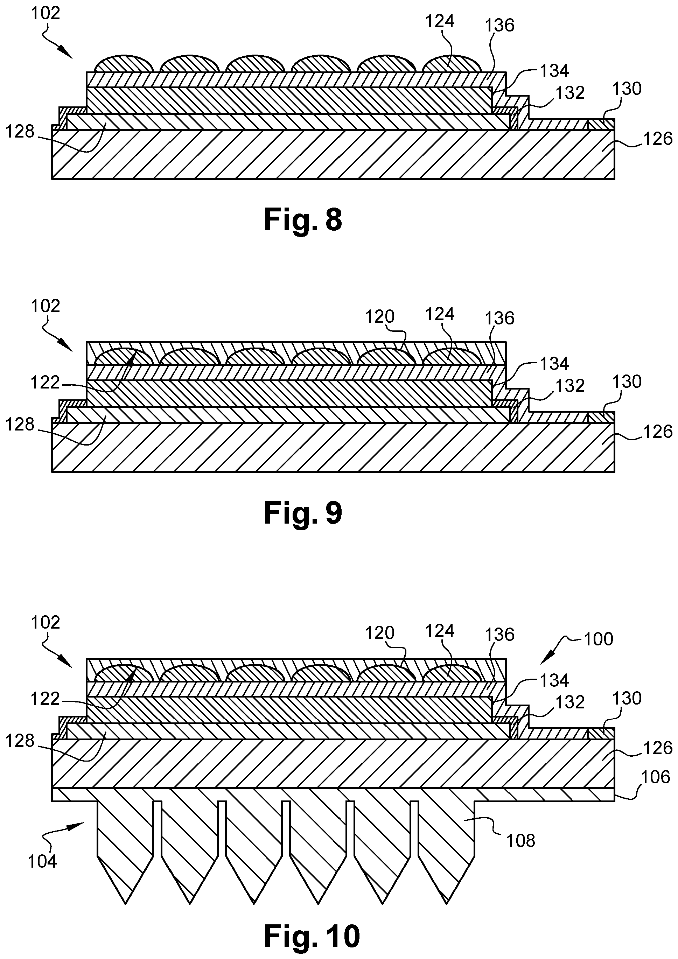

Une étape de fluage peut ensuite être mise en œuvre pour donner aux plots 138 la forme souhaitée et former ainsi les composants optiques 124 (voir figure 8).

La couche réflectrice 120 est ensuite réalisée, par exemple par dépôt, sur les composants optiques 124 et sur les parties de la deuxième électrode 136 non recouvertes par les composants optiques 124 (voir figure 9).

Le dispositif 100 est achevé en reportant la structure réalisée sur l'élément de guidage 104 comprenant, dans l'exemple de réalisation décrit, la base 106 et les microaiguilles 108. Ce report correspond, dans l'exemple décrit, à une solidarisation du substrat 126 contre la base 106 (voir figure 10).

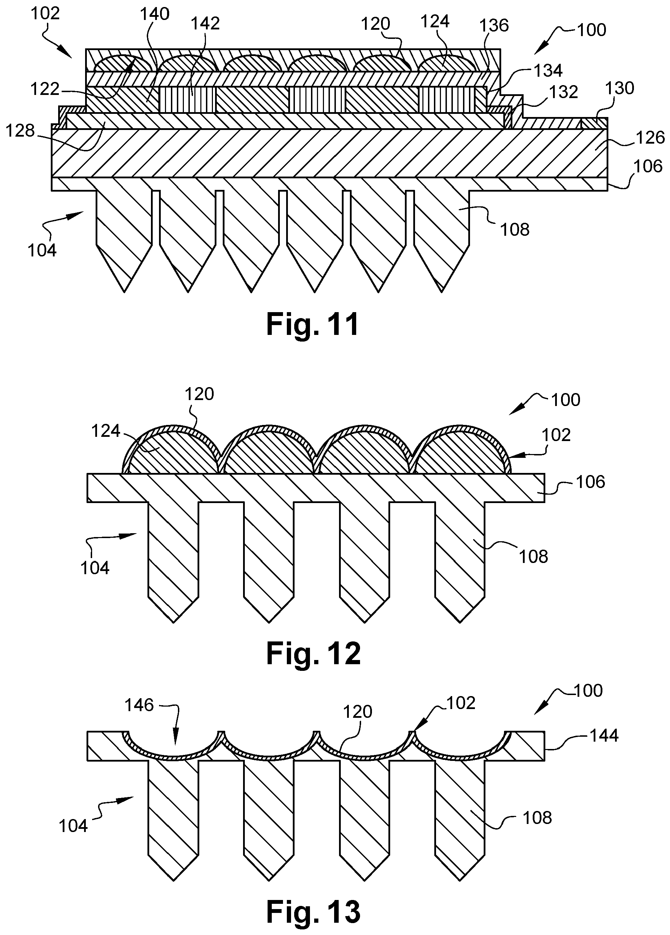

Dans une variante, la source lumineuse 102 peut être réalisée telle qu'elle comporte plusieurs parties distinctes configurées pour émettre des lumières de longueurs d'onde différentes et qui sont disposées chacune en regard d'au moins une des microaiguilles 108. Un exemple de réalisation d'un dispositif 100 selon une telle variante est représenté sur la figure 11. Sur cette figure, la ou les couches émissives 134 comportent des premières parties émissives 140 et des deuxièmes parties émissives 142 disposées de manière alternée les unes à côté des autres sur la première électrode 128. Selon un exemple de réalisation, les premières parties émissives 140 peuvent être configurées pour émettre une lumière rouge (qui a pour propriété de bien traverser l'épiderme), et les deuxièmes parties émissives 142 peuvent être configurées pour émettre une lumière bleue (qui a pour propriété d'être bien absorbée par le principe actif utilisé dans les traitements par photothérapie).

En variante, la source lumineuse 102 peut être configurée pour émettre des longueurs d'onde différentes des exemples décrits ci-dessus, et/ou un plus grand nombre de longueurs d'onde différentes.

Un exemple de dispositif émissif lumineux 100 selon un deuxième mode de réalisation est décrit ci-dessous en lien avec la figure 12.

Dans ce deuxième mode de réalisation, le dispositif 100 comporte l'élément de guidage optique 104 formé de la base 106 et des microaiguilles 108. Les composants optiques 124 sont disposés sur la base 106 de l'élément de guidage optique 104.

Contrairement au premier mode de réalisation dans lequel la source lumineuse 102 est disposée entre l'élément de guidage optique 104 et les composants optiques 124, et avec la couche réflectrice 120 formée au-dessus des composants optiques 124, les composants optiques 124 sont ici disposés sur l'élément de guidage optique 104, avec la source lumineuse 102 disposée sur les composants optiques 124. Autrement dit, les composants optiques 124 sont ici disposés entre l'élément de guidage optique 104 et la source lumineuse 102. En outre, dans ce deuxième mode de réalisation, la couche réflectrice 120 correspond à l'une des électrodes de l'OLED formant la source lumineuse 102 et correspond à celle formant une couche externe de la source lumineuse 102 (c'est-à-dire celle qui n'est pas disposée directement contre les composants optiques 124). Pour que cette électrode forme la couche réflectrice 120, et donc la surface réfléchissante 122, cette électrode comporte par exemple au moins l'un des matériaux suivants : Ag, Al, Au, Cr, etc., ainsi qu'une épaisseur suffisante pour que cette couche soit opaque et réfléchissante.

Dans ce deuxième mode de réalisation, les différentes couches de la source lumineuse 102 (électrodes et couche(s) émissive(s)) sont déposées de manière conforme sur les surfaces non-planes formées par les composants optiques 124. Par rapport à une source lumineuse 102 plane telle que précédemment décrite en lien avec le premier mode de réalisation, la surface émissive de la source lumineuse 102 selon le deuxième mode de réalisation est plus importante, pour un encombrement donné sur la surface de l'élément de guidage optique 104, ce qui permet d'augmenter la quantité de lumière envoyée dans l'élément de guidage optique 104. Sur l'exemple de la figure 12, en réalisant les composants optiques 124 tels qu'ils soient disposés les uns à côté des autres en se touchant, la quantité de lumière émise peut être environ six fois plus importante que dans le cas d'une source lumineuse 102 plane.

Dans l'exemple de réalisation représenté sur la figure 12, les composants optiques 124 sont de forme concave, ce qui implique que la surface réfléchissante formée par la couche réflectrice 120 est également concave.

Dans une variante représentée sur la figure 13, les composants optiques 124 du dispositif 100 sont formés à partir d'une couche de matériau 144 au moins partiellement transparente à la lumière destinée à être émise par la source lumineuse 102, cette couche de matériau 144 comprenant des creux 146 disposés à l'aplomb des microaiguilles 108. Ainsi, ces creux 146 forme des surfaces convexes telles que les surfaces réfléchissantes formées par la couche réflectrice 120 en regard des microaiguilles 108 soient également convexes. En outre, dans cette variante, l'élément de guidage optique 104 ne comporte pas la base 106 mais uniquement les microaiguilles 108. Comme dans l'exemple précédemment décrit, cette variante permet d'obtenir une surface émissive de la source lumineuse 102 plus importante, pour un encombrement donné sur la surface de l'élément de guidage optique 104, et donc d'augmenter la quantité de lumière envoyée dans l'élément de guidage optique 104.

Les différentes variantes précédemment décrites pour le premier mode de réalisation peuvent s'appliquer à ce deuxième mode de réalisation.

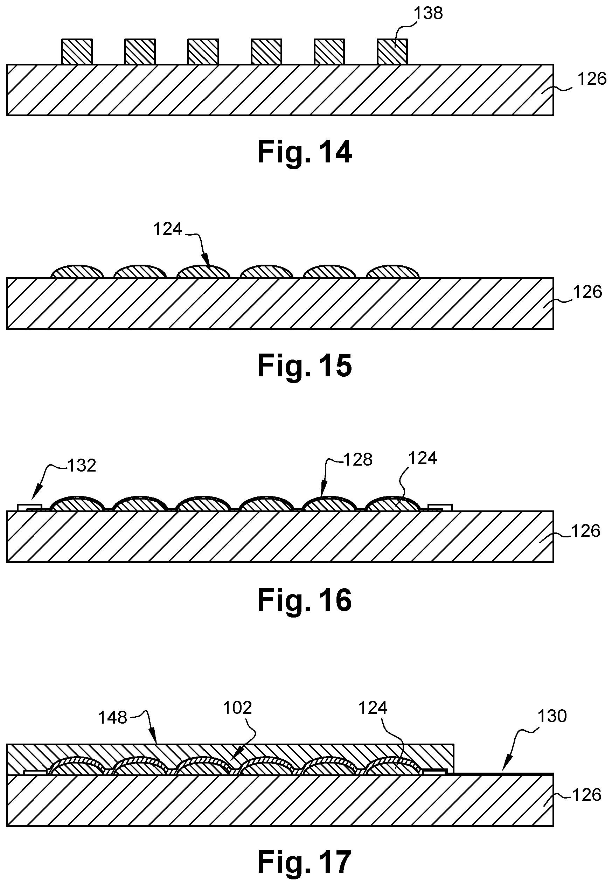

Un exemple de procédé de réalisation du dispositif 100 selon le deuxième mode de réalisation est décrit ci-dessous en lien avec les figures 14 à 17.

Dans cet exemple, les composants optiques 124 sont tout d'abord réalisés en déposant sur le substrat 126 au moins une couche de matériau transparent ou semi-transparent destinée à la réalisation des composants optiques 124. Cette couche de matériau comporte par exemple une résine transparente ou semi-transparente. Des étapes de photolithographie et de développement peuvent ensuite être mises en œuvre pour former les plots 138 de matériau par exemple similaires à ceux précédemment décrits pour le premier mode de réalisation. La structure obtenue à ce stade du procédé est représentée sur la figure 14.

Une étape de fluage peut ensuite être mise en œuvre pour former les composants optiques 124 (voir figure 15).

La première électrode 128 est ensuite réalisée, par exemple par dépôt, sur les composants optiques 124 ainsi que sur des parties du substrat 126, entre et à côté des composants optiques 124. Comme dans le premier mode de réalisation, la portion isolante 132 est ensuite réalisée (voir figure 16).

La source lumineuse 102 est ensuite achevée en déposant la ou les couches émissives 134, puis la deuxième électrode 136 (avec, dans l'exemple décrit, la réalisation du plot de contact 130). L'ensemble est ensuite recouvert d'une couche d'encapsulation 148 pouvant être transparente ou opaque (voir figure 17).

Le dispositif 100 est ensuite achevé en reportant le substrat 126 sur un élément de guidage optique 104, par exemple similaire à l'un des exemples précédemment décrits.

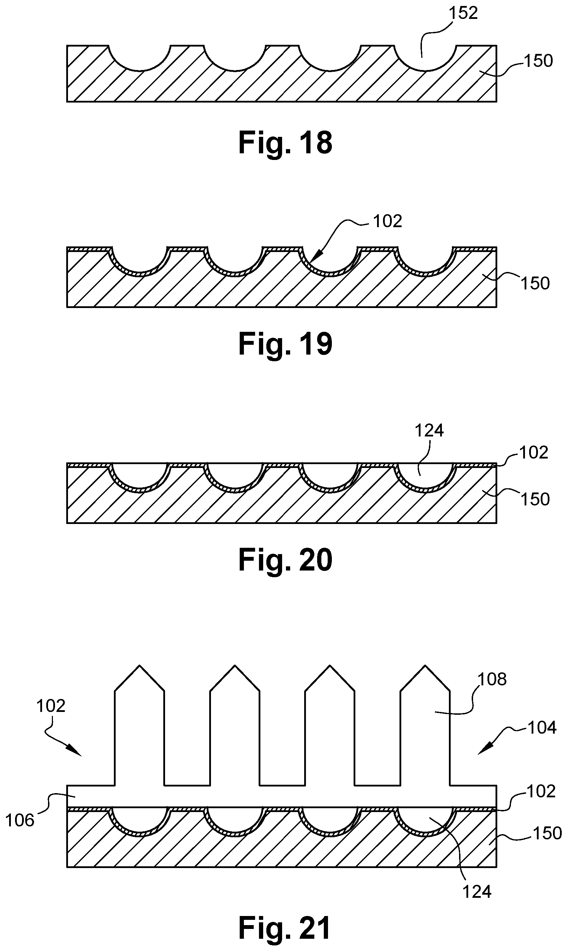

Selon un autre exemple de réalisation décrit en lien avec les figures 18 à 21, le dispositif 100 peut être réalisé à partir d'un substrat opaque 150.

Le substrat opaque 150 est tout d'abord gravé au niveau d'une de ses faces en réalisant des creux 152 formant des surfaces convexes (voir figure 18). Une première électrode est ensuite déposée sur la face du substrat 150 précédemment gravée, et également dans les creux 152, de manière à former l'anode et la surface réfléchissante. Une ou plusieurs couches émissives sont ensuite déposées sur la première électrode. Une deuxième électrode est ensuite déposée sur la ou les couches émissives, achevant la réalisation de la source lumineuse 102. Une couche d'encapsulation transparente ou semi-transparente est enfin déposée sur la deuxième électrode (voir figure 19). Le volume restant des creux 152 qui n'est pas occupé par les couches déposées pour former la source lumineuse 102 et par la couche d'encapsulation est rempli d'un matériau formant les composants optiques 124 (voir figure 20). L'ensemble réalisé est ensuite reporté, par exemple par collage, sur un substrat comprenant le ou les éléments de guidage optique 104 (voir figure 21).

Dans tous les modes de réalisation, le dispositif 100 permet d'optimiser l'injection de lumière dans l'élément de guidage optique 104 grâce à l'utilisation judicieuse de la surface réfléchissante 122 non plane et qui, combinée à une source lumineuse 102 émettant de la lumière à la fois du côté de l'élément de guidage optique 104 et du côté de la surface réfléchissante 102, permet d'augmenter la quantité de lumière envoyée vers l'élément de guidage optique 104 étant donné que la lumière émise du côté de la surface réfléchissante 122 est récupérée et réfléchie en direction de l'élément de guidage optique 104 grâce aux propriétés de réflexion et de focalisation de la lumière de la surface réfléchissante 122.

Divers modes de réalisation et variantes ont été décrits. La personne du métier comprendra que certaines caractéristiques de ces divers modes de réalisation et variantes pourraient être combinées, et d'autres variantes apparaîtront à la personne du métier.

Enfin, la mise en œuvre pratique des modes de réalisation et variantes décrits est à la portée de la personne du métier à partir des indications fonctionnelles données ci-dessus. Par exemple, la nature précise des étapes de dépôt et de gravure mises en œuvre peut être choisie en fonction notamment du ou des matériaux à déposer ou à graver, ainsi que des épaisseurs des matériaux à déposer ou à graver.

## Revendications

1. Dispositif émissif lumineux (100) comprenant au moins :
- un élément de guidage optique (104) ;
- une source lumineuse (102) au moins partiellement transparente à au moins une lumière destinée à être émise par la source lumineuse (102), et configurée pour émettre la lumière au moins du côté d'une première face (116) disposée en regard de l'élément de guidage optique (104) et du côté d'une deuxième face (118) opposée à la première face (116) ;
- une couche réflectrice (120) disposée du côté de la deuxième face (118) de la source lumineuse (102) ;
- un composant optique (124) disposé entre la couche réflectrice (120) et l'élément de guidage optique (104) et au moins partiellement transparent à la lumière destinée à être émise par la source lumineuse (102);
et dans lequel la couche réflectrice (120) forme au moins une surface réfléchissante (122) conforme à une surface non plane du composant optique (124) sur laquelle la couche réflectrice (120) est disposée.

2. Dispositif émissif lumineux (100) selon la revendication 1, dans lequel la source lumineuse (102) comporte au moins une diode électroluminescente organique.

3. Dispositif émissif lumineux (100) selon la revendication 2, dans lequel la couche réflectrice (120) est l'une des électrodes de la diode électroluminescente organique.

4. Dispositif émissif lumineux (100) selon l'une quelconque des revendications précédentes, dans lequel la surface réfléchissante (122) comporte au moins une partie concave ou convexe.

5. Dispositif émissif lumineux (100) selon l'une quelconque des revendications précédentes, dans lequel la surface réfléchissante (122) forme au moins un miroir sphérique ou conique ou hyperbolique.

6. Dispositif émissif lumineux (100) selon l'une quelconque des revendications précédentes, dans lequel la couche réflectrice (120) comporte au moins une couche métallique et/ou au moins un miroir de Bragg.

7. Dispositif émissif lumineux (100) selon l'une quelconque des revendications précédentes, comportant en outre au moins un substrat (126) au moins partiellement transparent à la lumière destinée à être émise par la source lumineuse (102) et disposé entre l'élément de guidage optique (104) et la source lumineuse (102).

8. Dispositif émissif lumineux (100) selon l'une quelconque des revendications précédentes, dans lequel l'élément de guidage optique (104) comporte au moins une microaiguille (108) au moins partiellement transparente à la lumière destinée à être émise par la source lumineuse (102), ou au moins un guide d'onde.

9. Dispositif émissif lumineux (100) selon la revendication 8, dans lequel l'élément de guidage optique (102) comporte une base (106) au moins partiellement transparente à la lumière destinée à être émise par la source lumineuse (102) et plusieurs microaiguilles (108) au moins partiellement transparentes à la lumière destinée à être émise par la source lumineuse (102) et comprenant chacune une première extrémité (112) solidaire de la base (106), la base (106) étant disposée entre la source lumineuse (102) et les microaiguilles (108).

10. Dispositif émissif lumineux (100) selon la revendication 8, dans lequel l'élément de guidage optique (104) comporte plusieurs microaiguilles (108) au moins partiellement transparentes à la lumière destinée à être émise par la source lumineuse (102), et dans lequel le composant optique (124) comporte une couche de matériau (144) au moins partiellement transparent à la lumière destinée à être émise par la source lumineuse (102), la couche de matériau (144) comprenant des creux (146) alignés avec les microaiguilles (108).

11. Dispositif émissif lumineux (100) selon l'une des revendications 9 ou 10, dans lequel la source lumineuse (102) comporte plusieurs parties (140, 142) distinctes configurées pour émettre des lumières de longueurs d'onde différentes, chacune desdites parties (140, 142) étant disposée en regard d'au moins une des microaiguilles (108).

12. Dispositif émissif lumineux (100) selon l'une quelconque des revendications 9 à 11, dans lequel la source lumineuse (102) est configurée pour émettre une partie de la lumière entre les microaiguilles (108).

13. Dispositif émissif lumineux (100) selon l'une quelconque des revendications 9 à 12, dans lequel au moins une partie de la surface réfléchissante (122) est configurée pour réfléchir une partie de la lumière émise du côté de la deuxième face (118) de la source lumineuse (102) entre les microaiguilles (108).

14. Procédé de réalisation d'un dispositif émissif lumineux (100), comprenant au moins :
- réalisation d'au moins un élément de guidage optique (104) ;
- réalisation d'au moins une source lumineuse (102) au moins partiellement transparente à au moins une lumière destinée à être émise par la source lumineuse (102), et configurée pour émettre la lumière au moins du côté d'une première face (116) disposée en regard de l'élément de guidage optique (104) et du côté d'une deuxième face (118) opposée à la première face (116) ;
- réalisation d'au moins une couche réflectrice (120) disposée du côté de la deuxième face (118) de la source lumineuse (102) ;
- réalisation d'au moins un composant optique (124) disposé entre la couche réflectrice (120) et l'élément de guidage optique (104) et au moins partiellement transparent à la lumière destinée à être émise par la source lumineuse (102) ;
et dans lequel la couche réflectrice (120) est réalisée telle qu'elle forme au moins une surface réfléchissante (122) conforme à une surface non plane du composant optique (124) sur laquelle la couche réflectrice (120) est disposée.

15. Procédé de réalisation selon la revendication 14, dans lequel :
- la source lumineuse (102) et la couche réflectrice (120) sont réalisés sur l'élément de guidage optique (104), ou
- la source lumineuse (102) et la couche réflectrice (120) sont réalisées sur un substrat (126) au moins partiellement transparent à la lumière destinée à être émise par la source lumineuse (102), le substrat (126) étant solidarisé ensuite à l'élément de guidage optique (104), ou
- la source lumineuse (102), la couche réflectrice (120) et le composant optique (124) sont réalisés sur un substrat (150) opaque à la lumière destinée à être émise par la source lumineuse (102), le composant optique (124) étant solidarisé ensuite à l'élément de guidage optique (104).
